# EUROPEAN PATENT APPLICATION

(11) **EP 1 811 032 A1**
(43) Date of publication of application: **25.07.2007**
(21) Application number: 06075148.4
(22) Date of filing: 23.01.2006
(51) Int. Cl.: C12N 15/12, C07K 14/47, C07K 16/18, G01N 33/53, C12Q 1/68

(54) **C-terminal exon 5.4 of RUNX1/AML1**

(71) Applicant: Julius-Maximilians-Universität Würzburg, 97070 Würzburg (DE)
(72) Inventor: Gattenlöhner, Stefan, 97080 Würzburg (DE); Marx, Alexander, 97080 Würzburg (DE)
(74) Representative: Elbel, Michaela

(57) **Abstract**

The present invention relates to a novel C-terminal exon of RUNX1/AML1, its nucleic acid sequence, its peptide and a full length amino acid sequence comprising the novel C-terminal exon. Furthermore, the invention is directed to an antibody against the C-terminal exon of RUNX1/AML1, a pharmaceutical composition for the treatment of various diseases, among others various tumours and the use of the C-terminal exon for the manufacture of a medicament. The C-terminal exon may also be used for the inhibition of cellular growth and/or induction of apoptosis. Furthermore, the invention relates to a method and a kit for diagnosing a disease, both using the C-terminal exon.

## Description

### Field of the invention

The present invention relates to the technical field of tumour therapy, in particular to the diagnosis, prophylaxis and therapy of RUNX1/AML1 related diseases by applying a novel C-terminal exon of the transcription factor RUNX1/AML1.

The most common cause of chronic heart failure is coronary artery disease, which results in left ventricular dysfunction. The morphological changes of the heart in chronic heart failure due to coronary artery disease have been termed ischemic cardiomyopathy (ICM).

Among the earliest events during ischemia induced ventricular dysfunction, the renin-angiotensin system and secretion of atrial natriuretic peptide (ANP) are activated. In addition, in the endothelin system, cytokines such as IL-1, IL-6, and tumour necrosis factor-α, stress-proteins, and anti-oxidants change their expression pattern. However, these changes generally are not characteristic for ICM.

To identify differentially overexpressed genes in ICM compared to normal hearts, in previous studies the inventors used a PCR-based technique to construct a subtracted cDNA library. It was shown that strong overexpression of CD56/NCAM and the transcription factor RUNX1/AML1 is a highly sensitive and characteristic marker of cardiomyocytes within or adjacent to scars in ICM compared to normal hearts, while at most slight overexpression is observed in CCM, hypertrophic obstructive cardiomyopathy (HOCM), and myocarditis, including sarcoidosis. This molecular response to ischemic heart damage appears to be phylogenetically conserved, because analogous alterations occurred in an experimental rat model of ischemic heart disease compared to normal and spontaneously hypertensive rats (Gattenlohner et al., 2003, 2004).

RUNX1/AML1 is essential for normal embryonal and foetal development. In contrast, stem cells in bone marrow do not, or only in part, depend on RUNX1/AML1. In adults, RUNX1/AML1 is important for the T-cell, B-cell and megakaryocyte development (Yamaguchi et al., 2004).

RUNX1/AML1 expression is different in stem cells with an exclusive haematopoietic potential of (RUNX1+FLK1(-)) and stem cells with haematopoietic and endothelial potential (RUNX1 (-), FLK-1+), the later ones also being called haemangioblasts.

RUNX1/AML1 is expressed in normal thymus cortex, while RUNX3 is expressed in the medulla (Woolf et al., 2003). It has been shown that RUNX1/AML1 and RUNX3 are repressors of CD4 expression in intrathymic T-cell development, and they are important for the establishment of CD8+ T-cells. Therefore, they might be important for a physiological immunodeficiency in elderly patients (Woolf et al., 2003, Telfer et al., 2004; Taniuchi et al., 2002).

Additionally, RUNX1/AML1 is essential for the early B-cell development. RUNX1/AML1 is expressed in proliferative chondrocytes, in osteoblasts in bone formation, in the thyroid gland, in the lung, in the skin and in normal epithelial cells of the trachea. RUNX1/AML1 is also important for the early development of the skeleton (Lian et al., 2003; Lian et al., 2003a; 2003b; 2003c).

RUNX1/AML1 is important for the development of cholinergic motor neurones in the afterbrain. RUNX genes are target genes of TGF-β and BMPs (Ito et al., 2003). RUNX1/AML1 regulates TIMP1 (MMP inhibitor) and plays an important role in fibrosis, e.g. in the development of chronic liver cirrhosis (Bertrand-Philippe et al., 2004). Additionally, RUNX1/AML1 regulates the expression of PKC-β and GATA3 and thereby influences the development of a TH₂ immune response. Finally, RUNX1/AML1 is co-regulated with mSin3A and CBF-β.

RUNX1/AML1 has been shown to play an important role in various neoplasia and tumours. RUNX1/AML1 is associated with translocations, point mutations and amplifications in various kinds of leukaemia, and RUNX1/AML1 is involved in about 25 % of all human leukaemia (Yamaguchi et al., 2004). RUNX1/AML1 seems to play a role in lymphoma and in plasmocytomas, since T-cell lymphoma in mice express RUNX1/AML1 (Wotton et al., 2002). RUNX1/AML1 is also expressed in melanoma (Perry et al., 2002a; 2002b), breast (Dairkee et al., 2004) and prostate cancer (Fowler et al., 2006).

Additionally, RUNX1/AML1 and dependent genes can be regarded as risk factors for autoimmune diseases like rheumatoid arthritis, psoriasis and diabetes mellitus (type 1 diabetes) (Tokuhiro et al., 2003; Yamada et al., 2004; Nielsen et al., 2003).

CD56/NCAM is a downstream target of RUNX1/AML1. CD56/NCAM regulates the growth of neurons and the flexibility of neuronal synapses (Panicker et al., 2003; Berezin et al., 2004). CD56/NCAM plays a role in the regulation of cell matrix interaction, in particular in the metastasis of primary tumours (Christofori et al., 2003; Cavallaro et al., 2001). CD56/NCAM overexpression is typical for the loss of nerves in skeletal muscles or scar development after heart diseases (Gattenlohner et al., 2003).

Even though many correlations between various kinds of tumours and genes overexpressed in certain tissues have been demonstrated during the last decade, there is still a need for a pharmaceutical composition for the efficient treatment and/or prophylaxis of certain kinds of tumours, cardiomyopathy, myocarditis, sarcoidosis and and autoimmune diseases.

### Summary of the invention

The present invention is directed to a C-terminal exon of RUNX1/AML1, wherein the exon is defined by the nucleic acid sequence of AAC AGT TGT GAT TAC TTC AGG TTT CAC CAG ATG CCT TGA (SEQ ID NO:1) and/or functional variants thereof.

Furthermore, the present invention relates to a peptide of a C-terminal exon of RUNX1/AML1, wherein the peptide is defined by the amino acid sequence of NSCDYFRFHQMP (SEQ ID NO:2) and/or functional variants thereof.

Moreover, the present invention comprises a nucleic acid sequence comprising an untranslated region (UTR) and a C-terminal exon of RUNX1/AML1, wherein the nucleic acid sequence is defined by SEQ ID NO:3.

The present invention also comprises an antibody which is directed against a peptide sequence of the C-terminal exon of RUNX1/AML1 of SEQ ID NO:2 and/or functional variants thereof.

It is a further object of the present invention to provide a pharmaceutical composition comprising a nucleic acid sequence and/or a peptide sequence of the C-terminal exon of RUNX1/AML1 of SEQ ID NO:1, 2, 3, an antibody against SEQ ID NO:2 and/or functional variants thereof.

The present invention is also directed to the use of a nucleic acid sequence and/or a peptide sequence of the C-terminal exon of RUNX1/AML1 of SEQ ID NO:1, 2, 3, an antibody against SEQ ID NO:2 and/or functional variants thereof for the manufacture of a medicament and the use of a nucleic acid sequence and/or a peptide sequence of the C-terminal exon of RUNX1/AML1 of SEQ ID NO:1, 2, 3, an antibody against SEQ ID NO:2 and/or functional variants thereof for inhibition of cellular growth and/or induction of apoptosis.

Furthermore, the present invention relates to a method for diagnosing a disease in a subject comprising: a) assaying a sample from the subject for a nucleic acid sequence and/or a peptide sequence of the C-terminal exon of RUNX1/AML1 of SEQ ID NO:1, 2, 3, an antibody against SEQ ID NO:2 and/or functional variants thereof; and b) determining the level of the nucleic acid sequence and/or a peptide sequence of the C-terminal exon of RUNX1/AML1 of SEQ ID NO:1, 2, 3, an antibody against SEQ ID NO:2 and/or functional variants thereof, wherein an altered level compared to a control sample indicates the presence of the disease.

The present invention also concerns a kit for diagnosing a disease comprising a nucleic acid sequence and/or a peptide sequence of the C-terminal exon of RUNX1/AML1 of SEQ ID NO:1, 2, 3, an antibody against SEQ ID NO:2 and/or functional variants thereof.

Finally, it is also an object of the present invention to provide a method for treatment of a disease, comprising administering to a subject in need thereof a therapeutically effective amount of a nucleic acid sequence and/or a peptide sequence of the C-terminal exon of RUNX1/AML1 of SEQ ID NO:1, 2, 3, an antibody against SEQ ID NO:2 and/or functional variants thereof.

Preferred diseases are indicated in the section "detailed description of the invention".

### Brief description of the drawings

Figure 1 shows a diagram with RUNX1/AML1 isoform 1 of the prior art and three new isoforms 2, 3 and 4 of the present invention.
Figure 2 shows a Western Blot analysis of the novel C-terminal exon 5.4 probed with the monoclonal antibody anti-RUNX1 C-ter EX 5.4.
Figure 3 shows the results of transfection experiments with different RUNX1/AML1 isoforms and luciferase as reporter gene expressed from the CD56/NCAM promoter.
Figure 4 shows a semi quantitative RT-PCR for CD56/NCAM after transfection of the cell line K562 with RUNX1/AML1 isoforms 1, 2, 3 and 4, a vector control (vec) and untransfected cells (blanc).
Figure 5 shows a semi quantitative RT-PCR for Granzyme after transfection of the cell line K562 with RUNX1/AML1 isoforms 1, 2, 3 and 4, a vector control (vec) and untransfected cells (blanc).
Figure 6 shows a semi quantitative RT-PCR for GM-CSF after transfection of the cell line K562 with RUNX1/AML1 isoforms 1, 2, 3 and 4, a vector control (vec) and untransfected cells (blanc).
Figure 7 shows a semi quantitative RT-PCR for p21 after transfection of the cell line K562 with RUNX1/AML1 isoforms 1, 2, 3 and 4, a vector control (vec) and untransfected cells (blanc).
Figure 8 shows a semi quantitative RT-PCR for CD3/gamma after transfection of the cell line K562 with RUNX1/AML1 isoforms 1, 2, 3 and 4, a vector control (vec) and untransfected cells (blanc).
Figure 9 shows an immunofluorescence (Cy3) of K562 cells after transfection with RUNX1/AML1 isoform 1.

### Detailed description of the invention

The present invention relates to a C-terminal exon of RUNX1/AML1, wherein the exon is defined by the nucleic acid sequence of AAC AGT TGT GAT TAC TTC AGG TTT CAC CAG ATG CCT TGA (SEQ ID NO:1) and/or functional variants thereof and a peptide of a C-terminal exon of RUNX1/AML1, wherein the peptide is defined by the amino acid sequence of NSCDYFRFHQMP (SEQ ID NO:2) and/or functional variants thereof.

The inventors have demonstrated that CD56/NCAM, a neural cell adhesion molecule and a member of the immunoglobuline super family is overexpressed in chronic ischemic heart failure compared to normal hearts. While searching for the factors which are responsible for the overexpression of CD56/NCAM, the inventors identified the transcription factor RUNX1/AML1 by its specific binding to the CD56/NCAM promoter in an electro mobility shift assay. In a Western Blot assay with a commercially available antibody against the N-terminal region of RUNX1/AML1 a 30 kd protein signal was surprisingly detected in addition to the expected signal at around 50 kd. The identity of the 30 kd polypeptide was unknown. The inventors successfully isolated the corresponding cDNA by a rapid amplification of cDNA ends (RACE-PCR). A subsequent radioactively labelled colony hybridisation of a cDNA gene bank of a human heart with a known RUNX1/AML1 probe led to the isolation of three novel, so far unknown isoforms of RUNX1/AML1. The different isoforms are characterised by exchanges at the C-terminal exon of RUNX1/AML1. The novel exon was designated as the C-terminal exon 5.4 of RUNX1/AML1 = C-ter EX 5.4.

The isolation of this novel exon led to the isolation of new RUNX1/AML1 isoforms, for which the novel exon 5.4 is required. The novel isoform of the protein RUNX1/AML1 confers a new function in comparison to the prior art isoform of RUNX1/AML1. The novel isoforms comprising the novel exon 5.4 at the C-terminus show a dominant negative transactivating function since this isoform is significantly down regulated in human ischemic cardiomyopathy (ICM) in comparison to normal heart tissue.

The dominant negative function of the novel exon 5.4 is a particular advantage of the present invention, since the novel exon can be used for the therapeutic and/or prophylactic treatment of diseases associated with RUNX1/AML1 target genes, and the novel C-terminal exon 5.4 is particularly useful for the therapy of various diseases indicated further below.

As used herein, the term "functional variants" of the nucleic acid sequence coding for the novel C-terminal exon of RUNX1/AML1 refers to any nucleic acid sequence which is at least 95 % identical to SEQ ID NO:1. Also encompassed is the complementary nucleic acid sequence. In particular, nucleotide exchanges which do not lead to a change of the respective amino acid (silent exchanges) are also encompassed. As used herein, the term "functional variants" of a peptide of the novel C-terminal exon of RUNX1/AML1 refers to a polypeptide sequence which is at least 95 % identical to SEQ ID NO:1. In particular, conservative amino acid exchanges are enclosed. Furthermore, modifications like methylation etc. can be done without leaving the scope of the term "functional variants".

The invention further comprises a nucleic acid sequence comprising an untranslated region (UTR) and a C-terminal exon of RUNX1/AML1, wherein the nucleic acid sequence is defined by SEQ ID NO:3. The natural context of the C-terminal exon 5.4 is indicated in diagram 1 below. The first 36 nucleotides of the open reading frame are the coding sequence of the novel exon RUNX1/AML1 (indicated in bold letters), followed by a stop codon *(TAG in italic).* An untranslated region (UTR) of approximately 670 to 700 nucleotides follows the stop codon. A polyadenylation site (TAAATAA underlined) and a further stretch of a 40 nucleotide A-rich region form the end of SEQ ID NO:3.

### Diagram 1: C-terminal exon 5.4 in its natural context (SEQ ID NO:3)

AACAGTTGTGATTACTTCAGGTTTCACCAGATGCCTTAG*StopCTGCTGT
TCAGAGCTTCACAGAATGCCTTCTAAGAGCTTCACAACGGCAGTACTA

TTGACATTTGGGGCCAAATAATTCTTTGTTGGGGGGGTCTGTCCTGA
GCATCGGAGAATATTTAGCAGCATCCTTGGCCTCTAGATGCCAATAGC
ATCCCCCTCACCCCAGTTAGGACAATAAAAATGTTTCCAGACATTGC
CAAATTTCCCAAGGGGCAAAACTGTCCCCAGGTGAGAACCACTGCCTT
CCAGTTGTTCTTGGGTGGTACAGTAAAATAGGAAAACCAACTTAAGTG
CTTGGTTTTCACATTACTTTTGGCCATTAATGCCAATATTTTCATGGTAC
ATTCTGCTCCAAGGCATTGAGAGTAGCACAGGTATCTTGGCATCTTGT
TTAGCTTGTGGTTTCTAGAAGCTTATCCCTCAAGAACAAAGGCACTTGT
CAGCTTTTACTGATTGTTCTCAGGGCACCCCTCTGCACACCGGCTACG
GTCTTCACCTGAAATAATGCCTGTTCTCCTTCAAATGTTCCTGCTATGG
GTCTATCCTTTGGAATTTATTACATAAGTCTTTTGACTTGGTCATCACC
CAAGATCAGCACCTTATAGTCCAAGAACCCTAGGGGCCAACTCCTCCA
GCTGACTGGTGTTCAGATGGTAGGGTGACTTTTTAAATAA polyadenylation site
AAGGAATGAGTAACAAAAAA AAAAAAAAAAAAAAAAAAAAAAAA

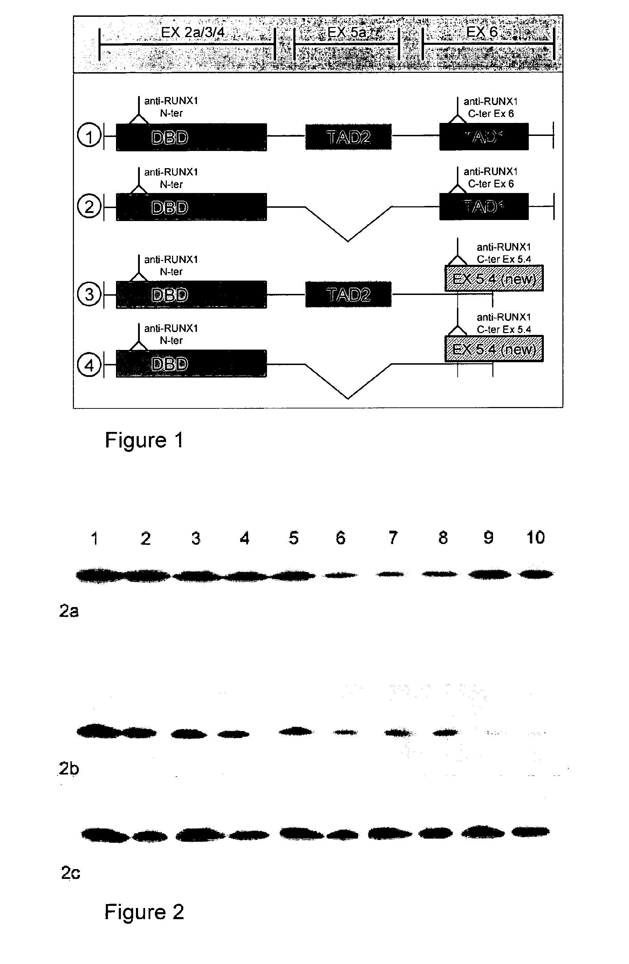

The inventors successfully developed an anti-RUNX1/AML1 C-terminal exon 5.4 polyclonal antibody in a rabbit, which was used for Western Blots (see Figure 2). Thus, the present invention also provides an antibody which is directed against a peptide sequence of the C-terminal exon of RUNX1/AML1 of SEQ ID NO:2 and/or functional variants thereof. The antibody may be a monoclonal or a polyclonal antibody.

It is another aspect of the present invention to provide a pharmaceutical composition comprising a nucleic acid sequence and/or a peptide sequence of the C-terminal exon of RUNX1/AML1 of SEQ ID N0:1, 2, 3, an antibody against SEQ ID NO:2 and/or functional variants thereof. The pharmaceutical composition may further comprise a pharmaceutically acceptable carrier and/or additive. The appropriate concentration of the therapeutic agent, in particular the nucleic acid sequence and/or the peptide sequence of the C-terminal exon of RUNX1/AML1 might be dependent on the particular agent. The therapeutically effective dose has to be compared with the toxic concentrations; the clearance rate, the metabolic products, the solubility and the formulation may play an important role. Therapeutic efficiency and toxicity of compounds can be determined by standard pharmaceutical procedures in cell cultures or experimental animals e.g. ED50 (the dose therapeutically effective in 50 % of the population) and LD50 (the dose lethal to 50 % of the population). The dose ratio between therapeutic and toxic effects is the therapeutic index, and it can be expressed as the ratio of LD50/ED50.

In a particularly preferred embodiment of the present invention the composition is used for the therapeutic and or prophylactic treatment of diseases associated with RUNX1/AML1 target genes. Examples of RUNX1/AML1 target genes are MDR1 (multi drug resistant 1), CD4, CD3, GM-CSF (granulocyte macrophage colony stimulating factor), TCR (T cell receptor) α chain, TCR-β chain, TCR-γ chain, TCR-δ chain, IL3, Granzyme B, M-CSF receptor, MPO (myeloperoxidase), p14, p21, CD11a, CD36, CD53, UBP43 (ubiquitin binding protein) and/or CD56/NCAM. The disease, which can be advantageously therapeutically and/or prophylactically addressed is selected from the group consisting of congestive cardiomyopathy, hypertrophic obstructive cardiomyopathy, myocarditis, sarcoidosis, hypoplastic haematopoietic disease, autoimmune diseases, degenerative and neoplastic diseases of the central and peripheral nervous system, diseases of the vascular system, developmental diseases of the gut, degenerative, traumatic and developmental diseases of the skeletal system, skeletal and heart muscle atrophy and eye diseases.

A hypoplastic haematopoietic disease may be an aplastic anaemia or single or multiple cytopenias. Diabetes type 1 is a particularly preferred autoimmune disease to be treated by the novel exon 5.4 of RUNX1/AML1. The inventors have further shown that PML, i.e. a factor interacting with RUNX1/AML1, is most important for central tolerance based on regulating gene expression in the thymus. Diseases of the vascular system include the therapeutic promotion or inhibition of angiogenesis. Particular examples are the therapy of ischemic alterations concerning the brain, heart, gut or limbs, implying the therapeutic aim to promote angiogenesis, or the inhibition of vessel formation, which is a conceptual aim in anti-angiogenesis for the treatment of virtually all tumours with a prominent vascular structure, degenerative, traumatic and developmental diseases of the skeletal system include bones, joints, cartilage and teeth. The skeletal or heart muscle atrophy also involves states of denervation, inactivation, inflammatory disease or a consequence of a tumour or an infection. Eye diseases include, in particular, diseases of the retina.

The composition of the present invention is particularly useful for the therapeutic and/or prophylactic treatment of a tumour. Therefore, in a preferred embodiment of the present invention the tumour is selected from the group consisting of lymphoma, leukaemia, rhabdomyosarcoma, adenocarcinoma, tumour of the duodenum, testis, lymph node, kidney, thymus, primary and secondary myelodysplastic syndromes, myeloma, plasmacytoma, melanoma, tumours of the peripheral and central nervous system and breast and prostate cancer. The primary and secondary myelodysplastic syndromes relate, in particular, to therapy-related myelodysplastic syndromes.

In yet another embodiment of the present invention the use of a nucleic acid sequence and/or a peptide sequence of the C-terminal exon of RUNX1/AML1 of SEQ ID NO:1, 2, 3, an antibody against SEQ ID NO:2 and/or functional variants thereof for the manufacture of a medicament is provided. The medicament is particularly useful for the therapeutic and/or prophylactic treatment of diseases associated with RUNX1/AML1 target genes. Such genes have been exemplarily described in this application and it is referred to the respective paragraphs in the above specification. Additionally, preferred diseases have already been described and, as the invention is particularly useful for the therapeutic and/or prophylactic treatment of a tumour, reference is made to the above examples for particularly useful tumours.

Another embodiment of the present invention relates to the use of a nucleic acid sequence and/or a peptide sequence of the C-terminal exon of RUNX1/AML1 of SEQ ID N0:1, 2, 3, an antibody against SEQ ID NO:2 and/or functional variants thereof for inhibition of cellular growth and/or induction of apoptosis. Apoptosis or programmed cell death is a well known instrument of organisms to eliminate specific cells. Further, the use of a nucleic acid sequence and/or a peptide sequence of the C-terminal exon of RUNX1/AML1 of SEQ ID NO:1, 2, 3, an antibody against SEQ ID NO:2 and/or functional variants for the elucidation of changes of the cell states in terms of differentiation and trans-differentiation is encompassed.

In a particularly preferred embodiment of the present invention a method is provided for diagnosing a disease in a subject comprising: a) assaying a sample from the subject for a nucleic acid sequence and/or a peptide sequence of the C-terminal exon of RUNX1/AML1 of SEQ ID NO:1, 2, 3, an antibody against SEQ ID NO:2 and/or functional variants thereof; and b) determining the level of the nucleic acid sequence and/or a peptide sequence of the C-terminal exon of RUNX1/AML1 of SEQ ID NO:1, 2, 3, an antibody against SEQ ID NO:2 and/or functional variants thereof, wherein an altered level compared to a control sample, indicates the presence of the disease. Specific examples of diseases are indicated above.

Additionally, the invention relates to a kit for diagnosing a disease comprising a nucleic acid sequence and/or a peptide sequence of the C-terminal exon of RUNX1/AML1 of SEQ ID N0:1, 2, 3, an antibody against SEQ ID NO:2 and/or functional variants thereof. Specific examples of diseases are indicated above.

Finally, the invention provides a method for the treatment of a disease comprising administering to a subject in need thereof a therapeutically effective amount of a nucleic acid sequence and/or a peptide sequence of the C-terminal exon of RUNX1/AML1 of SEQ ID NO:1, 2, 3, an antibody against SEQ ID NO:2 and/or functional variants thereof. Specific examples of diseases are indicated above.

### Best mode to carry out the invention

Figure 1 is referred to in the following which shows a schematic representation of the RUNX1/AML1 isoform 1 of the prior art (1) and the novel isoforms 2, 3 and 4, (2), (3) and (4), respectively. All isoforms share the DNA binding domain (DBD) which also comprises the RUNX domain. The C-terminal exon of isoforms 1 and 2 is different to the C-terminal exon of isoforms 3 and 4, and the novel exon in isoforms 3 and 4 is indicated as exon 5.4 (EX 5.4 new). The prior art C-terminal exon is called exon 6. Additionally, isoforms 2 and 4 do not harbour the penultimate exon, so called exon 5a, of isoforms 1 and 3. Isoforms 2 and 4 are also called splice variants. The splice variant isoform is known from mice but before the invention, it has not been identified in humans. Isoforms 2 and 4, due to the lack of the transactivation domain TAD2 in exon 5a, show a dominant negative influence on the CD56/NCAM promoter, which is the main promoter of an important RUNX1/AML1 target gene. In this respect, isoform 4, which harbours the novel exon 5.4, has an unexpected function.

The novel exon 5.4 is situated between the known exon 5.3 and the last exon 6. Due to this position, the inventors suggested naming the novel exon "exon 5.4". Exon 5.4 comprises a nucleic acid sequence of approximately 760 base pairs with an open reading frame (ORF) of 36 base pairs, coding for 12 amino acids and an untranslated region (UTR) of approximately 724 base pairs.

Using these 12 amino acids which code for the novel exon peptide 5.4, the inventors successfully developed a specific polyclonal antibody, named anti-RUNX1 C-ter EX5.4, which detects a prominent 30 kd signal in recombinant control extracts as well as in whole protein extracts of various samples of human heart muscles. This signal is successfully suppressed by homologue recombinant bacterially expressed protein.

In Figure 2 a Western Blot analysis with the novel polyclonal antibody anti-RUNX1 C-ter EX5.4 is shown. In panel 2a a whole cell extract of human heart muscle tissue was used for the detection of a specific signal (30 kd) with the monoclonal antibody. A more prominent expression was shown in normal heart tissue (lanes 1 to 5) in comparison to reduced expression in ischemic heart tissue (lanes 6 to 10). In panels 2b and 2c a specific and unspecific competition of the antibody binding with homologue recombinant bacterially expressed protein is shown, respectively. The polyclonal antibody anti-RUNX1 C-ter EX5.4 was incubated with a specific (2b) and an unspecific (2c) competitor which was recombinant GST/RUNX1 EX5.4-fusion protein and GST-protein alone, respectively. The polyclonal antibody anti-RUNX1 C-ter EX5.4 was pre-incubated with increasing concentrations of competitors (lanes 1/2: 0 mg; lanes 3/4: 50 mg; lanes 5/6: 100 mg; lanes 7/8: 250 mg and lanes 9/10: 500 mg) and subsequently used for the detection of a total protein extract of human heart muscle tissue. As can be derived from panel 2b, the competition was specific and prominent with respect to antibody binding with increasing concentrations of the competitor while the unspecific competitor in panel 2c did not lead to any significant change in antibody binding.

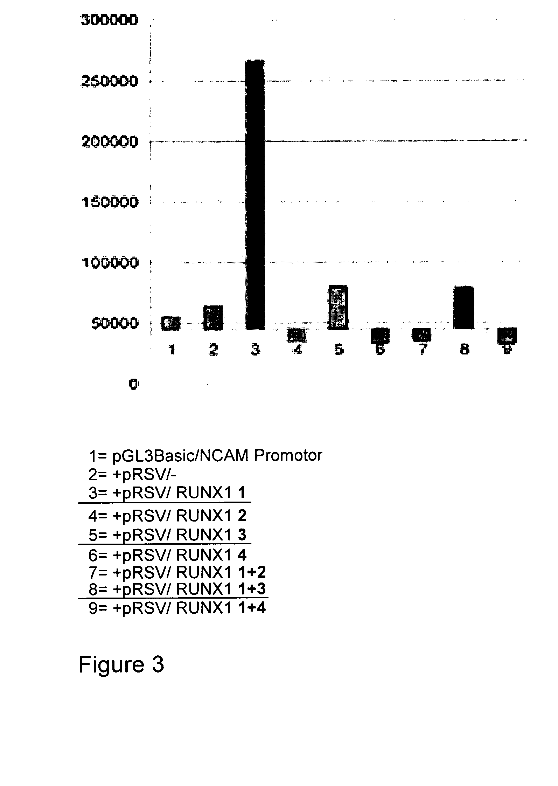

In Figure 3 the results of experiments with different RUNX1/AML1 isoforms are shown. RUNX1/AML1 isoforms 1 to 4 were coupled with luciferase as a reporter gene, which was expressed from the CD56/NCAM promoter. Control transfections merely show a very low luciferase activity (1 = vector construct; 2 = expression vector pRSV without insert). The prior art isoform 1 (No. 3) of RUNX1/AML1 shows a significant transactivating function, while the novel isoforms 2 and 4, both harbouring the novel exon 5.4 (No. 4 and No. 6, respectively), show a dominant negative action which is demonstrated by the drop in luciferase activity below the background level. Interestingly, isoform 3 (No. 5) shows a low transactivation function, and isoform 3 is able to inhibit the activating function of isoform 1, when co-incubated with isoform 1 (No. 8).

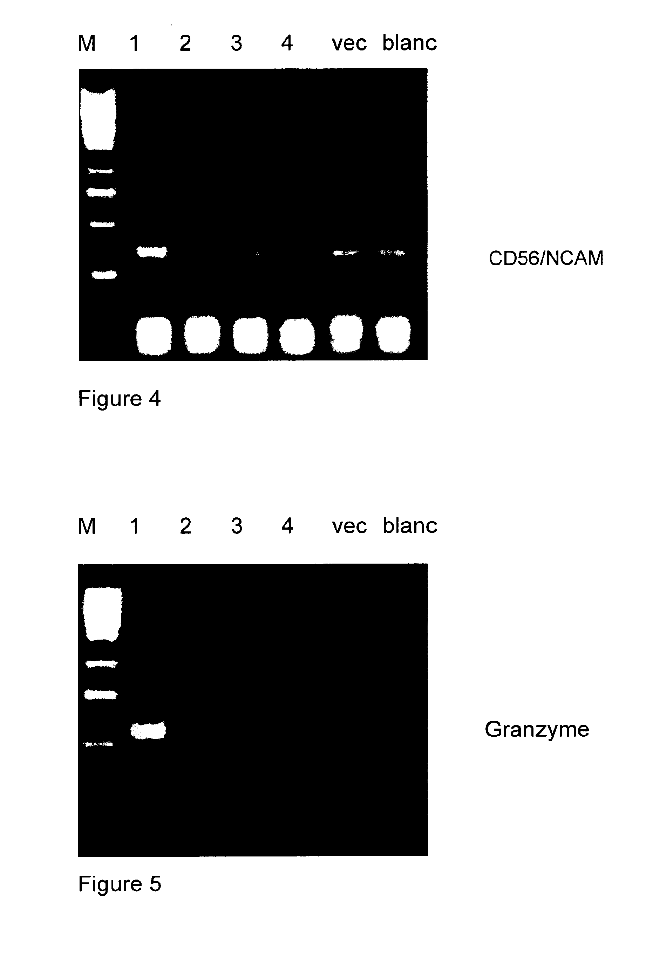

The inventors also tested several downstream targets of RUNX1/AML1, for which semi quantitative RT-PCRs after transfection of the cell line K562 with RUNX1/AML1 isoforms 1, 2, 3 and 4 were performed. Figure 4 shows a semi quantitative RT-PCR for CD56/NCAM in which isoforms 1 and 3 show a prominent signal, while isoforms 2 and 4, both being devoid of the transactivating domain 2 (TAD2), show, at best, a very weak signal. It was surprisingly discovered that the combination of the novel exon 5.4 and the lack of the transactivation domain TAD2 leads to complete abolishment of the signal. As a control, the vector (vec) and untransfected cells (blanc) were used.

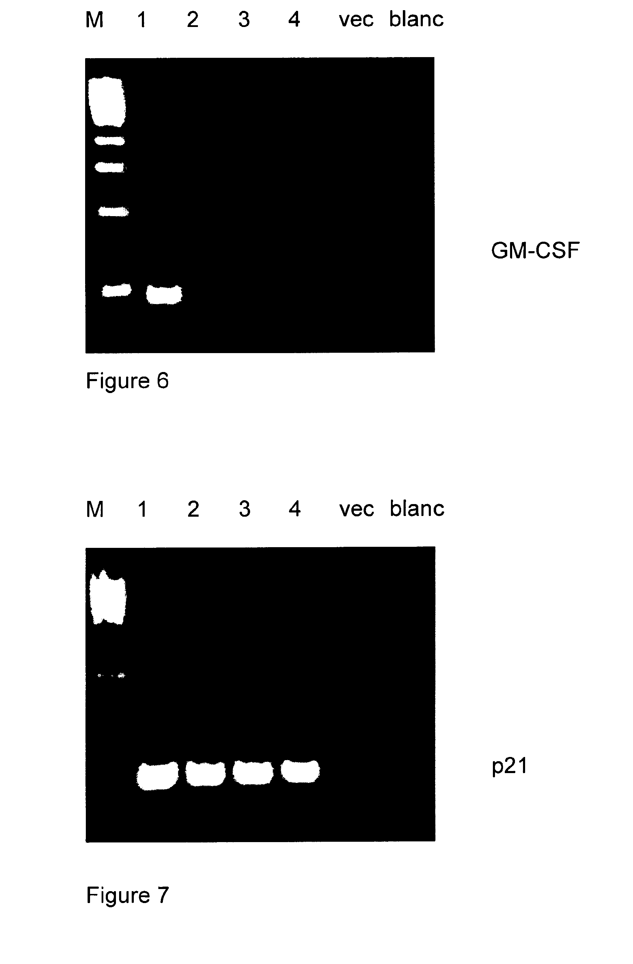

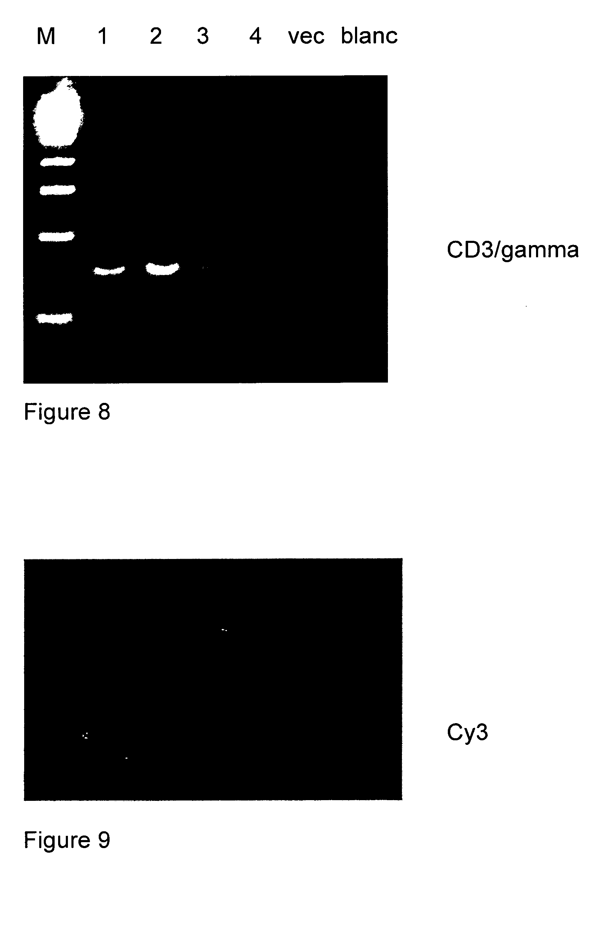

For the following experiments, the same semi quantitative RT-PCR conditions were employed for Granzyme (Figure 5), GM-CSF (Figure 6), p21 (Figure 7) and CD3/gamma (Figure 8).

In Figure 9 immunofluorescence (Cy3) of K562 cells after transfection with RUNX1/AML1 isoform 1 is shown. A polyclonal rabbit anti-RUNX1/AML1 antibody was used in a 1:100 dilution. As a secondary antibody, a Cy3 labelled goat antirabbit secondary antibody was used. The magnification is 400 fold. Almost the same transfection efficiencies were obtained when isoforms 2, 3 and 4 of RUNX1/AML1 were used. The transfection efficiency was almost 50 %.

The inventors describe here, for the first time, a novel and, up to now, unknown C-terminal exon of RUNX1/AML1 which is expressed as a 30 kd isoform in Western Blots. This novel isoform is the decisive regulating factor of the expression of CD56/NCAM because the 30 kd isoform can successfully inhibit the well known 50 kd isoform of RUNX1/AML1. The novel exon is relevant for the transcription of additional, functionally relevant genes, as demonstrated in the examples with CD56/NCAM, Granzyme, GM-CSF, p21 and CD3/gamma. The novel exon and the novel "inactivating" isoform of RUNX1/AML1 is relevant for the function of various genes, which play an important role in the normal and disturbed developmental processes in organ systems, e.g. developmental and neural biology, haematology and immunology, cardiovascular physiology and pathology, gut and liver functions, general metabolism and endocrinology as well as in tumour development and tumour progression and the development of autoimmune diseases. Therefore, the novel exon, its nucleotide sequence, the peptide, and the antibody as well as designed molecules based thereon show a broad variety of diagnostic and therapeutic applications.

In particular, the novel exon is expressed in various organs, like testis, lymph nodes, kidney, thymus and duodenum. Additionally, it shows a differential expression in various tumours, which are derived from these organs. Of particular advantage is such a differential expression in various lymphoma and leukaemia, but also in plasmocytoma and solid tumours like carcinoma.

Additionally, the novel isoform is the dominant isoform in hearts, in particular human hearts. The inventors have successfully shown in cell transfection assays that the novel 30 kd RUNX1/AML1 isoform has a constitutive and in comparison to the prior art 50 kd isoform only minor transactivating function. Additionally, the novel 30 kd isoform is much stronger than the 50 kd isoform of the prior art, since the strong transactivating function of the 50 kd isoform is completely abolished and inhibited by the 30 kd isoform. For these experiments the CD56/NCAM promoter was used as a test system (Figure 3). The inventors have additionally demonstrated in cell biological experiments, that the transfection of tumour cell lines with the novel RUNX1/AML1 isoform leads to an inhibition of growth and to the induction of apoptosis.

### Scientific, diagnostic and therapeutic application of RUNX1/AML1 exon 5.4

A promising diagnostic approach comprises the detection of normal or increased quantities of the exons by way of antibodies and/or molecular/nucleic acid probes. Also, the detection of low or lacking expression of the novel exon in virtually any tissue, including degenerative or inflammatory tissues and tumours is of particular advantage.

A very useful therapeutic approach comprises the substitution of an absolute or relative lack of the novel exon or the protein in tissues or tumours by targeted molecular manipulation or gene therapy. Furthermore, a relevant overexpression of activated RUNX1/AML1 isoforms may be compensated by therapy with the inhibitory, novel isoform of exon 5.4. Another therapeutic approach comprises the inhibition of abnormal, relative or absolute overexpression of the new isoform of the RUNX1/AML1 gene product by a gene therapeutic intervention with inhibitory or competitive nucleic acid sequences or peptides.

Examples of successful diagnostic, prophylactic and/or therapeutic approaches include the diagnosis and therapy of leukaemia, myelodysplastic syndromes, lymphoma and plasmocytoma or myeloma and other tumours with overexpression of the well known RUNX1/AML1 isoforms of the prior art, reduced expression of the novel RUNX1/AML1 isoform and the imbalance between the known and the new isoforms.

Additionally, diagnosis and therapy of disorders of blood development, e.g. hypoplastic or aplastic disorders or thrombocytopenia is possible. Furthermore, the prevention and therapy of fibrosis, e.g. hypertrophic scars, keloids, liver cirrhosis, lung fibrosis and myocardial fibrosis is possible. The inventors also envisage the diagnostic, prophylactic and/or therapeutic treatment of autoimmune diseases, the prevention of ischemic myocardial diseases after heart failure and the diagnosis and therapy of neurodegenerative disorders, in which RUNX1/AML1 is involved.

In particular, the novel exon/protein is useful in all applications, which are based on the manipulation of the RUNX1/AML1 target gene CD56/NCAM. The best example is the successful treatment of neurodegenerative diseases, the manipulation of CD56/NCAM expression in tumours with a CD56 hyperexpression (e.g. plastocytoma, melanoma, neuroendocrine tumours, many sarcoma, leukaemia and lymphoma).

### References

- 1.: Berezin, V. and E. Bock, NCAM mimetic peptides: Pharmacological and therapeutic potential. J Mol Neurosci, 2004. 22(1-2): p. 33-39.
- 2.: Bertrand-Philippe, M., et al., Regulation of tissue inhibitor of metalloproteinase 1 gene transcription by RUNX1 and RUNX2. J Biol Chem, 2004. 279(23): p. 24530-9.
- 3.: Cavallaro, U., et al., N-CAM modulates tumour-cell adhesion to matrix by inducing FGF-receptor signalling. Nat Cell Biol, 2001. 3(7): p. 650-7.
- 4.: Christofori, G., Changing neighbours, changing behaviour: cell adhesion molecule-mediated signalling during tumour progression. EMBO J, 2003. 22(10): p. 2318-23.
- 5.: Dairkee, SH. et al., A molecular 'signature' of primary breast cancer cultures; patterns resembling tumor tissue. BMC Genomics, 2004; 5(1):47.
- 6.: Fowler, M. et al., RUNX1 (AML-1) and RUNX2 (AML-3) cooperate with prostate-derived Ets factor to activate transcription from the PSA upstream regulatory region. J Cell Biochem. 2006; 97(1):1-17.
- 7.: Gattenlöhner S , W.C., Ertl G, Bültmann BD, and M.-H.H.-K.a.M. A, The overexpression of NCAM(CD56) in human hearts is specific for ischemic damage. 3. Posterpreis, Verhandlungsband der Deutschen Gesellschaft für Pathologie, 2004.
- 8.: Gattenlohner, S., et al., NCAM(CD56) and RUNX1(AML1) are up-regulated in human ischemic cardiomyopathy and a rat model of chronic cardiac ischemia. Am J Pathol, 2003. 163(3): p. 1081-90.
- 9.: Ito, Y. and K. Miyazono, RUNX transcription factors as key targets of TGF-beta superfamily signaling. Curr Opin Genet Dev, 2003. 13(1): p. 43-7.
- 10.: Lian, J.B., et al., Runx1/AML1 hematopoietic transcription factor contributes to skeletal development in vivo. J Cell Physiol, 2003a. 196(2): p. 301-11.
- 11.: Lian, J.B., et al., Runx2/Cbfa1 functions: diverse regulation of gene transcription by chromatin remodeling and co-regulatory protein interactions. Connect Tissue Res, 2003b. 44 Suppl 1: p. 141-8.
- 12.: Lian, J.B. and G.S. Stein, Runx2/Cbfa1: a multifunctional regulator of bone formation. Curr Pharm Des, 2003c. 9(32): p. 2677-85.
- 13.: Nielsen, C., et al., Association of a putative regulatory polymorphism in the PD-1 gene with susceptibility to type 1 diabetes. Tissue Antigens, 2003. 62(6): p. 492-7.
- 14.: Panicker, A.K., et al., Cellular signalling mechanisms of neural cell adhesion molecules. Front Biosci, 2003. 8: p. d900-11.
- 15.: Perry, C., A. Eldor, and H. Soreq, Runx1/AML1 in leukemia: disrupted association with diverse protein partners. Leuk Res, 2002a. 26(3): p. 221-8.
- 16.: Perry, C., et al., Complex regulation of acetylcholinesterase gene expression in human brain tumors. Oncogene, 2002b. 21 (55): p. 8428-41.
- 17.: Taniuchi, I., et al., Differential requirements for Runx proteins in CD4 repression and epigenetic silencing during T lymphocyte development. Cell, 2002. 111(5): p. 621-33.
- 18.: Telfer, J.C., et al., Localization of the domains in Runx transcription factors required for the repression of CD4 in thymocytes. J Immunol, 2004. 172(7): p. 4359-70.
- 19.: Tokuhiro, S., et al., An intronic SNP in a RUNX1 binding site of SLC22A4, encoding an organic cation transporter, is associated with rheumatoid arthritis. Nat Genet, 2003. 35(4): p. 341-8.
- 20.: Woolf, E., et al., Runx3 and Runx1 are required for CD8 T cell development during thymopoiesis. Proc Natl Acad Sci U S A, 2003. 100(13): p. 7731-6.
- 21.: Wotton, S., et al., Proviral insertion indicates a dominant oncogenic role for Runx1/AML-1 in T-cell lymphoma. Cancer Res, 2002. 62(24): p. 7181-5.
- 22.: Yamada, R., et al., SLC22A4 and RUNX1: identification of RA susceptible genes. J Mol Med, 2004. 82(9): p. 558-64.
- 23.: Yamaguchi, Y., et al., AML1 is functionally regulated through p300-mediated acetylation on specific lysine residues. J Biol Chem, 2004. 279(15): p. 15630-8.

## Claims

1. A C-terminal exon of RUNX1/AML1, wherein the exon is defined by the nucleic acid sequence of AAC AGT TGT GAT TAC TTC AGG TTT CAC CAG ATG CCT TGA (SEQ ID NO:1) and/or functional variants thereof.

2. A peptide of a C-terminal exon of RUNX1/AML1, wherein the peptide is defined by the amino acid sequence of NSCDYFRFHQMP (SEQ ID NO:2) and/or functional variants thereof.

3. A nucleic acid sequence comprising an untranslated region (UTR) and a C-terminal exon of RUNX1/AML1, wherein the nucleic acid sequence is defined by SEQ ID NO:3.

4. An antibody which is directed against a peptide sequence of the C-terminal exon of RUNX1/AML1 of SEQ ID NO:2 and/or functional variants thereof.

5. A pharmaceutical composition comprising a nucleic acid sequence and/or a peptide sequence of the C-terminal exon of RUNX1/AML1 of SEQ ID NO:1, 2, 3, an antibody against SEQ ID NO:2 and/or functional variants thereof.

6. The composition of claim 5 for the therapeutic and/or prophylactic treatment of diseases associated with RUNX1/AML1 target genes.

7. The composition of claim 5 or 6, wherein the RUNX1/AML1 target genes are selected from the group consisting of MDR1, CD4, CD3, GM-CSF, TCR-α chain, TCR-β chain, TCR-γ chain, TCR-δ chain, IL3, Granzyme B, M-CSF receptor, MPO, p14, p21, CD11a, CD36, CD53, UBP43 and CD 56/NCAM.

8. The composition of any of claims 5 to 7, wherein the disease is selected from the group consisting of congestive cardiomyopathy, hypertrophic obstructive cardiomyopathy, myocarditis, sarcoidosis, hypoplastic hematopoietic disease, autoimmune diseases, degenerative and neoplastic diseases of the central and peripheral nervous system, diseases of the vascular system, developmental diseases of the gut, degenerative, traumatic and developmental diseases of the skeletal system, skeletal and heart muscle atrophy and eye diseases.

9. The composition of any of claims 5 to 8 for the therapeutic and/or prophylactic treatment of a tumour.

10. The composition of claim 9, wherein the tumour is selected from the group consisting of lymphoma, leukaemia, rhabdomyosarcoma, adenocarcinoma, tumour of the duodenum, testis, lymph node, kidney, thymus, primary and secondary myelodysplastic syndromes, myeloma, plasmacytoma, melanoma, breast and prostate cancer and tumours of the peripheral and central nervous system.

11. Use of a nucleic acid sequence and/or a peptide sequence of the C-terminal exon of RUNX1/AML1 of SEQ ID NO:1, 2, 3, an antibody against SEQ ID NO:2 and/or functional variants thereof for the manufacture of a medicament.

12. Use of a nucleic acid sequence and/or a peptide sequence of the C-terminal exon of RUNX1/AML1 of SEQ ID NO:1, 2, 3, an antibody against SEQ ID NO:2 and/or functional variants thereof for inhibition of cellular growth and/or induction of apoptosis.

13. A method for diagnosing a disease as defined in any one of claims 6 to 10 in a subject comprising:
a) assaying a sample from the subject for a nucleic acid sequence and/or a peptide sequence of the C-terminal exon of RUNX1/AML1 of SEQ ID NO:1, 2, 3, an antibody against SEQ ID NO:2 and/or functional variants thereof; and
b) determining the level of the nucleic acid sequence and/or a peptide sequence of the C-terminal exon of RUNX1/AML1 of SEQ ID NO:1, 2, 3, an antibody against SEQ ID NO:2 and/or functional variants thereof, wherein an altered level compared to a control sample indicates the presence of the disease.

14. A kit for diagnosing a disease as defined in any one of claims 6 to 10, comprising a nucleic acid sequence and/or a peptide sequence of the C-terminal exon of RUNX1/AML1 of SEQ ID NO:1, 2, 3, an antibody against SEQ ID NO:2 and/or functional variants thereof.


SEQUENCE LISTING

<110> Julius-Maximilians-Universität Würzburg

<120> C-terminal Exon of RUNX/AML1

<130> U30007

<160> 3

<170> PatentIn version 3.3

<210> 1
<211> 39
<212> DNA
<213> Homo sapiens

<400> 1
aacagttgtg attacttcag gtttcaccag atgccttga 39

<210> 2
<211> 12
<212> PRT
<213> Homo sapiens

<400> 2

Asn Ser Cys Asp Tyr Phe Arg Phe His Gln Met Pro
1               5                   10

<210> 3
<211> 759
<212> DNA
<213> Homo sapiens

<400> 3
aacagttgtg attacttcag gtttcaccag atgccttagc tgctgttcag agcttcacag 60

aatgccttct aagagcttca caacggcagt actattgaca tttggggcca aataattctt 120

tgttgggggg ggtctgtcct gagcatcgga gaatatttag cagcatcctt ggcctctaga 180

tgccaatagc atccccctca ccccagttag gacaataaaa aatgtttcca gacattgcca 240

aatttcccaa ggggcaaaac tgtccccagg tgagaaccac tgccttccag ttgttcttgg 300

gtggtacagt aaaataggaa aaccaactta agtgcttggt tttcacatta cttttggcca 360

ttaatgccaa tattttcatg gtacattctg ctccaaggca ttgagagtag cacaggtatc 420

ttggcatctt gtttagcttg tggtttctag aagcttatcc ctcaagaaca aaggcacttg 480

tcagctttta ctgattgttc tcagggcacc cctctgcaca ccggctacgg tcttcacctg 540

aaataatgcc tgttctcctt caaatgttcc tgctatgggt ctatcctttg gaatttatta 600

cataagtctt ttgacttggt catcacccaa gatcagcacc ttatagtcca agaaccctag 660

gggccaactc ctccagctga ctggtgttca gatggtaggg tgacttttta aataaaagga 720

atgagtaaca aaaaaaaaaa aaaaaaaaaa aaaaaaaaa 759